# EUROPEAN PATENT SPECIFICATION

(11) **EP 1 183 383 B1**
(45) Date of publication and mention of the grant of the patent: **18.10.2006**
(21) Application number: 00940511.9
(22) Date of filing: 09.06.2000
(51) Int. Cl.: C12N 15/86, C12N 7/00, A61K 48/00, C12N 15/11

(54) **SIV-BASED PACKAGING-DEFICIENT VECTORS**
AUF SIV BASIERENDE VERPACKUNGSDEFIZIENTE VEKTOREN
VECTEURS A ENCAPSIDATION DEFICIENTE BASES SUR LE SIV

(30) Priority: 09.06.1999 GB 9913459; 19.07.1999 GB 9916911
(43) Date of publication of application: 06.03.2002
(73) Proprietor: CAMBRIDGE UNIVERSITY TECHNICAL SERVICES LIMITED, Cambridge CB2 1TS (GB)
(72) Inventor: LEVER, Andrew Dept. of Medicine Level 5 Box 157, Cambridge CB2 2QQ (GB); GREATOREX, Jane Dept. of Medicine Level 5 Box157, Cambridge CB2 2QQ (GB); MCCANN, Eamon CRC Institute for Cancer Studies, Birmingham B15 2TA (GB); BALAN, Preetha Fox Chase Cancer Center, Philadelphia, PA 19111 (US); THOMAS, Joan Dept. of Medicine Level 5 Box 157, Cambridge CB22QQ (GB)
(74) Representative: Roques, Sarah Elizabeth
(86) International application number: PCT/GB2000/002263
(87) International publication number: WO 2000/075351

(56) References cited:
- WO-A-97/48277
- WO-A-99/04026
- HARRISON GEOFFREY P ET AL: "Functional analysis of the core human immunodeficiency virus type 1 packaging signal in a permissive cell line." JOURNAL OF VIROLOGY, vol. 72, no. 7, July 1998 (1998-07), pages 5886-5896, XP002147391 ISSN: 0022-538X
- MCBRIDE M SCOTT ET AL: "The human immunodeficiency virus type 1 encapsidation site is a multipartite RNA element composed of functional hairpin structures." JOURNAL OF VIROLOGY, vol. 70, no. 5, 1996, pages 2963-2973, XP002147392 ISSN: 0022-538X
- BERKHOUT BEN ET AL: "Role of the DIS hairpin in replication of human immunodeficiency virus type 1." JOURNAL OF VIROLOGY, vol. 70, no. 10, 1996, pages 6723-6732, XP002147393 ISSN: 0022-538X
- MCCANN E M ET AL: "LOCATION OF CIS-ACTING SIGNALS IMPORTANT FOR RNA ENCAPSIDATION IN THE LEADER SEQUENCE OF HUMAN IMMUNODEFICIENCY VIRUS TYPE 2" JOURNAL OF VIROLOGY,US,THE AMERICAN SOCIETY FOR MICROBIOLOGY, vol. 71, no. 5, 1997, pages 4133-4137, XP000909426 ISSN: 0022-538X
- RIZVI TAHIR A ET AL: "Simian immunodeficiency virus RNA is efficiently encapsidated by human immunodeficiency virus type 1 particles." JOURNAL OF VIROLOGY, vol. 67, no. 5, 1993, pages 2681-2688, XP000946026 ISSN: 0022-538X cited in the application
- LEVER A M L ET AL: "GENE THERAPY: FROM BENCH TO BEDSIDE. LENTIVIRUS VECTORS FOR GENE THERAPY" BIOCHEMICAL SOCIETY TRANSACTIONS,GB,COLCHESTER, ESSEX, vol. 27, no. 6, December 1999 (1999-12), pages 841-847, XP000915750 ISSN: 0300-5127

## Description

### Field of the Invention

This invention relates to vectors and their use in gene transfer. The vectors are based on retroviruses, adapted so that they cannot package their own RNA, and which can be used as infectious agents to transfer foreign genes, e.g. for somatic gene therapy.

### Background of the Invention

Retroviruses are classified in several ways. They are divided into various groups on the basis of their morphology. These groups are A,B,C and D type viruses. They are also classified as belonging to one of three subfamilies, namely oncoviruses, spumaviruses and lentiviruses.

SIV belongs to the family of retroviruses designated C-type viruses. These are characterised by capsid assembly at the cell membrane, and include viruses of the lentivirus group, e.g. Human Immunodeficiency Virus (HIV).

Retroviruses are RNA viruses which replicate through a DNA proviral intermediate which is integrated in the genome of the infected host cell. The virion particle contains a dimer of positive-strand genomic RNA molecules. This genomic RNA is the full-length species transcribed from the proviral DNA by the host RNA polymerase II. A proportion of these full-length RNAs which encode the *gag* and *pol* genes of the virus is translated by the host cell ribosomes, to produce the structural and enzymic proteins required for production of virion particles. The provirus also gives rise to a variety of smaller singly and multiply-spliced mRNAs coding for the envelope proteins and, in the case of more complex retroviruses, a group of regulatory proteins. The genomic (and subgenomic) RNA molecules are structurally similar to cellular mRNAs in having a 5' m⁷G cap and a polyadenylated 3' tail.

A series of problems must be addressed for successful packaging of genomic RNA: The full-length RNA must be packaged preferentially over the spliced viral messages as it is the only one carrying the full complement of genetic information for the next generation of virions. The virus must also specifically select the genomic RNA against the enormous quantity and variety of physically similar host cell mRNAs as, unlike many other viruses, retroviruses do not generally arrest host RNA synthesis. There must be a mechanism whereby genomic RNA to be packaged is recognised such that a proportion is either protected from being translated and transported to an assembly site or is associated with the *gag* precursor polyprotein which it has encoded immediately after translation. Lastly, there is the stoichiometric problem of having to package the correct number of genomes in association with 3-4000 *gag* precursor proteins, adequate numbers of reverse transcriptase molecules, a protease, tRNA primers and, in some cases, multiple copies of regulatory proteins.

Packaging the genome thus entails problems of specificity of selection of RNA and also considerations of RNA compartmentalisation.

The virus overcomes these problems by the presence of *cis*-acting elements, i.e. "packaging signals", in the viral genomic mRNA. Studies on spontaneously arising and laboratory constructed viral mutants have confirmed that specific sequences are critical for RNA recognition and encapsidation. Linial *et al*, Cell 15:1371-1381 (1978); Mann *et al,* Cell 33:153-159 (1983); Watanabe *et al,* PNAS USA 79:5986-5990 (1979) and WO-A-9119798 disclose that deletions in the 5' untranslated leader sequence lead to defects in packaging in, respectively, Rous Sarcoma Virus (RSV), Moloney Murine Leukemia Virus (MoMLV), Spleen Necrosis Virus (SNV) and HIV.

Deletion mutants have defined sequences necessary for RNA packaging in several retroviruses. In some of these, the extent of the sequence sufficient for packaging has also been mapped. Implicit in the description of packaging signals and RNA secondary structure is the premise that, if this sequence is introduced into heterologous RNA then, theoretically, the heterologous RNA should be encapsidated by retroviral particles. Constraints on packaging include the theoretical one (for which Mann *et al*, J. Virol. 54:401-407 (1985), provide some circumstantial evidence) that sequences adjacent to the packaging signal (PSI) should not favour the formation of alternative secondary structures disrupting PSI. Additionally, the total length of RNA packaged is physically limited by the capacity of the virus to package RNA of a certain size. In HIV, proviral constructs incorporating heterologous genes have been shown by Terwilliger *et al,* PNAS USA 86:3857-3861 (1989), to lead to a replication defect when the total length of the viral RNA produced significantly exceeds that of the original virus. The replication defect is consistent with a declining efficiency of RNA packaging.

Nevertheless, there is significant variability between different viruses in the nature and site of their encapsidation sequences. The mechanism of RNA recognition is so poorly understood that theoretically it is not possible to make predictions of the exact site and nature of encapsidation sequences without experimental data.

The development of retroviral vector systems has been a direct development of the work described above. In these systems, a packaging-defective "helper" virus is used to generate particles which encapsidate a highly modified RNA genome (the vector). Watanabe *et al,* Mol. Cell Biol. 3:2241-2249 (1983), and Eglitis *et al,* BioTechniques 6:608-614 (1988), report that vectors containing a minimum of the viral long terminal repeats, the packaging signal and a primer-binding site together with a heterologous marker gene have been encapsidated into virion particles and transferred to the cells for which the parent virus is tropic. By this means, it has been possible to define the minimal sequence required for encapsidation of RNA into a virus particle.

Adam *et al*, J. Virol. 62:3802-3806 (1988), disclose that, for MoMLV, the sequence sufficient for packaging encompasses the 5' leader region first defined by deletion as leading to a packaging defect. No additional sequences were essential although *gag* sequences enhanced packaging of the vector.

WO-A-9119798 discloses that an HIV-based vector containing essentially only the 5' leader sequence as a potential packaging signal was reported to be successfully encapsidated by an HIV-based packaging system. This work has not yet been confirmed. Indeed, there has been failure to encapsidate HIV RNA containing only the 5' leader sequence.

Although prior suggestions (see Rizvi and Panganiban 1993. J. of Virol., 67:2681.) have been made for the approximate position of the packaging signal in SIV, by the methodology of using other viruses to package the SIV RNA, this work is fundamentally flawed for several reasons. Firstly, a considerable stretch of the SIV genome was included and, thus, there was no attempt to define the sequences sufficient for packaging. From this work, there would only be the most incomplete suggestion as to how to use SIV sequences to package heterologous genes. In addition, the region used contained many other important *cis*-acting sequences for SIV including the primer-binding site, splice donor and the complete 5' leader region. Deletion of this whole region from SIV in order to generate a packaging-defective virus would be completely unsuccessful because of the importance of other segments of this region for viral transcription, reverse transcription, splicing, etc. Thus, the work disclosed by Rizvi and Panganiban does not define sequences that are necessary or sufficient for SIV packaging to an extent which might be useful for development of SIV as a vector. Secondly, the concept of using packaging by a second virus for determining the specific packaging sequences has recently shown to be flawed. Kaye *et al* (1998) demonstrate, for example, that HIV-1 and HIV-2 show a non-reciprocal packaging relationship such that packaging of one genome by another virus does not imply that those sequences are the relevant ones for packaging of the viral genome by its own proteins. Neither does absence of packaging of one virus by another mean that sequences included in the RNA have no relevance for packaging when this occurs in the context of the virus's own proteins.

### Summary of the Invention

According to the present invention, a provirus is capable of producing SIV proteins but is not replication-competent because the RNA cannot be packaged into virions. Using this packaging-defective provirus vector, packaging-defective cell lines can be created and used to investigate the packaging mechanism of the virus and to develop strategies to interfere with this packaging mechanism. Virions produced by such packaging-negative proviruses may be used for vaccines and as a system for efficiently introducing a desired gene into a mammalian cell.

The present invention provides a Simian Immunodeficiency Virus (SIV) genome having a mutation within the packaging signal such that viral RNA is not packaged within an SIV capsid. The genome has a mutation and in particular a deletion in the region between the primer binding site and the 5' major splice donor site, preferably within the DIS structure. The mutation comprises deletion of
(a) a sequence of SEQ ID no 1, or
(b) a fragment thereof of 5 or more nucleotides in length, or
(c) a variant of either thereof.

In another aspect of the present invention, there is provided a viral vector comprising an SIV packaging signal and a heterologous gene capable of being expressed in the vector.

The invention also provides a process for producing an SIV virus encoding a heterologous gene, which process comprises infecting a host cell with a packaging defective SIV genome according to the invention and a viral vector according to the invention expressing a heterologous gene and having SIV packaging sequences sufficient to package the vector in an SIV capsid; and culturing the host cell. Viruses produced in accordance with the invention can be used to deliver the heterologous gene to a host cell, for example in a method of gene therapy, vaccination or in scientific investigation.

In another aspect of the present invention, an SIV packaging sequence is used in the treatment or prophylaxis of SIV or HIV infection.

### Description of the Figures

Figure 1A and Figure 1B - SIV based construct used for packaging studies and sites of deletions in the 5' leader.
Figure 2 - Riboprobes used to distinguish viral RNA from DNA and to identify spliced and unspliced viral RNA. Predicted fragment sizes protected are shown.
Figure 3 (a-e) - RNA folds showing predicted secondary structure of SIV_{mac} leader RNA. a) wild type b) with Δ1 mutation c) with Δ2 mutation d) with Δ3 mutation e) with Δ4 mutation. Positions of deletions are shown in a) by arrows and bold type. Shaded areas indicate sequences corresponding to the primer binding site, dimerisation initiation signal and *gag* initiation codon. Thick lines in b), c), d) and e) indicate position of the deleted sequence.

### Description of the Invention

SIV has several potential advantages as a retroviral vector, compared to other retroviruses which have already been used and are being developed as gene vectors for potential use in humans. In particular:
1. It is a virus of the lentivirus family and, therefore, has the ability to enter and efficiently integrate the genes it is carrying into a cell which is not undergoing mitosis. Thus, unlike vectors based on murine or avian retroviruses, a vector system based on SIV will be able to target important tissues such as neurons, cardiac muscle and liver cells which divide rarely or never in the adult human.
2. There is no evidence that SIV is pathogenic in humans, unlike the human immunodeficiency viruses type 1 and type 2 which are being developed as lentivirus vectors for gene transfer in humans. Using HIV-1 or HIV-2 as vectors involves the chance, however remote, that during manufacture or passage or possibly in an infected individual, the vector sequences may recombine with sufficient other viral sequences to recreate a full-length infectious and pathogenic human immunodeficiency virus.
3. SIV has a genome of greater than 8 kilobases which appears to be largely redundant for packaging function and which could, thus, be replaced by the desired genetic sequences. Most cDNA copies of RNAs coding for genes which are candidates for gene therapy would fit into a viral genome of these dimensions.
4. The molecular biology and virus assembly functions of SIV are well understood.
5. SIV can be pseudotyped with alternative viral envelopes. Thus, it has the ability to deliver genes to cells bearing the CD4 antigen using the SIV envelope itself. Alternatively, new envelopes can be adjoined to the virus in order to direct its tropism to any desired cell shape.
6. There is no apparent detectable homology within endogenous human retroviral sequences, thus minimising the potential for recombination between therapeutic vectors and endogenous sequences that would lead to replication-competent virus arising *in vitro.*
7. There is no evidence that SIV encapsidates heterologous viral RN sequences such as the VL30 family encapsidated by murine retroviral vectors and transmitted to all cells transduced by murine retroviral vector systems.

The present invention is based on studies of the molecular biology and replication of wild-type and mutant SIV and defective virus constructs made therefrom (vectors), in which segments of the 5' prime leader region have been deleted in order to localise the cis acting signals involved in viral RNA packaging in SIV. Signals both necessary and sufficient for packaging of RNA into a virion particle have been defined. It appears that the localisation of signals necessary and sufficient for RNA packaging differs from other viruses previously studied, and that the site of these signals could not have been predicted by direct analogy to other retroviruses and study of their packaging mechanism. Vectors have been constructed which themselves are replication-defective but whose RNA can be packaged in *trans* by wild-type virus and delivered to target cells for which SIV itself is tropic by infection. These vectors integrate into the target cell chromosome and express the target gene efficiently enough in the case of antibiotic resistance markers to produce target cells which themselves are antibiotic-resistant.

In particular, it has been discovered that it is possible to make SIV packaging-defective vectors. It has been found that the region between the primer-binding site and the 5' major splice donor in SIV contains sequences necessary for efficient packaging of SIV RNA into virions. In addition, it has been found that the region between the 5' major splice donor and the *gag* initiation codon contains a second and less important region, important but not essential for packaging of SIV RNA into virions. One can prepare a vector comprising a packaging-defective SIV provirus, wherein the vector contains a nucleotide sequence which corresponds to a sufficient number of nucleotides from an SIV genome to express desired SIV products, but does not correspond to a sufficient number of nucleotides corresponding to the region between the primer-binding site and the 5' major splice donor or between the splice donor and the *gag* initiation codon to efficiently package SIV RNA (the packaging sequence).

These sequences preferably correspond to the genome of SIV. The term corresponds means that conservative additions, deletions and substitutions are permitted. The primer-binding site (23 bp) and the 5' major splice donor are respectively numbered 121-143 and 295-296 in the genomic nucleotide sequence where the transcript start site is defined as 1.

In particular, an SIV genome as used herein refers to the viral RNA derived from an SIV. The simian immunodeficiency viruses (SIV) of the invention may be derived from any SIV strain, or derivatives thereof. Derivatives preferably have at least 70% sequence homology to the SIV genome, more preferably at least 80%, even more preferably at least 90 or 95%. Other derivatives which may be used to obtain the viruses of the present invention include strains that already have mutations in some SIV genes such as a mutation in the nef gene as described in Rud et al 1994 J. Gen Virol 75, 529-543. Other mutations may also be present as set out in more detail below. The position of the primer binding site and 5' major splice donor site can readily be established by one skilled in the art by reference to the published SIV sequences or for example by aligning a variant SIV to the sequences set out and described herein.

In accordance with one aspect of the invention a SIV genome has a mutation within the packaging signal such that the SIV RNA is not packaged within the SIV envelope protein or capsid. Preferably such an SIV genome is capable of producing an SIV capsid. In an alternative aspect of the invention, a vector is provided which comprises an SIV packaging signal and a heterologous gene. References to the packaging sequences to be deleted from the SIV genome below also relate to a description of those sequences to be incorporated in a vector for packaging with the SIV capsid.

Preferably, the packaging defective genome does not contain the SIV packaging sequences corresponding to the segments immediately downstream of the primer-binding site and just upstream of the 5' major splice donor and/or those immediately downstream of splice donor and immediately upstream of the *gag* gene. Typically, the vector may contain nucleotides ranging from about 20 bases of the primer-binding site to about 80 bases downstream of the primer-binding site and still be packaging-deficient and/or about 20 bases downstream of major splice donor to 70 bases downstream.

Preferably the packaging sequence absent from the vector comprises part of the region between the primer binding site and the 5' major splice site. In one embodiment, the packaging sequence absent from the vector in this region contains the 85-base nucleotide sequence shown herein as SEQ ID No. 1, i.e.
AGAACTCCTGAGTACGGCCTGAGTGAAGGCAGTAAGGGCGGCAGGAACC AACCACGACGGAGTGCTCCTATAAAGGCGCAGGTCG.

The mutation may comprise a deletion of (a) the sequence of SEQ ID no 1, or (b) a fragment thereof of 5 or more nucleotides in length, or (c) a variant of either thereof. A variant of the sequence identified in SEQ ID No 1 is a corresponding sequence derived from a variant SIV genome, which may be identified for example by identifying the major 5' splice donor site, primer binding site or gag initiation codon and aligning the sequence of the variant to SEQ ID No 1 to identify the corresponding sequence of the variant SIV genome to SEQ ID No 1.

In a preferred embodiment, the packaging signal comprises part or all of the region of the genome 5' to the major splice donor site, for example a region commencing 90, 80, 70, 60 or 50 nucleotides upstream of (5' to) the 5' major splice donor site, extending to 40, 30, 20 or 10 nucleotides upstream of (5' to) the 5' major splice donor site. The mutation may comprise deletion or mutation within this region, for example to modify or delete 5, 10, 15, 20, 30, 40, 50 or 60 or more nucleotides from this region. For example the packaging deletion may comprise nucleotides 53 to 85 of SEQ ID No 1.

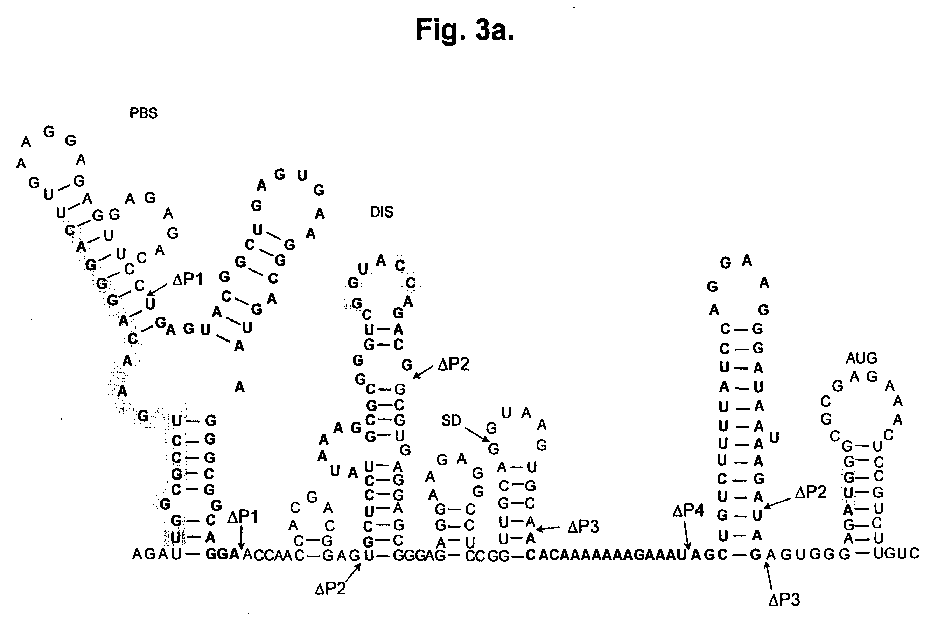

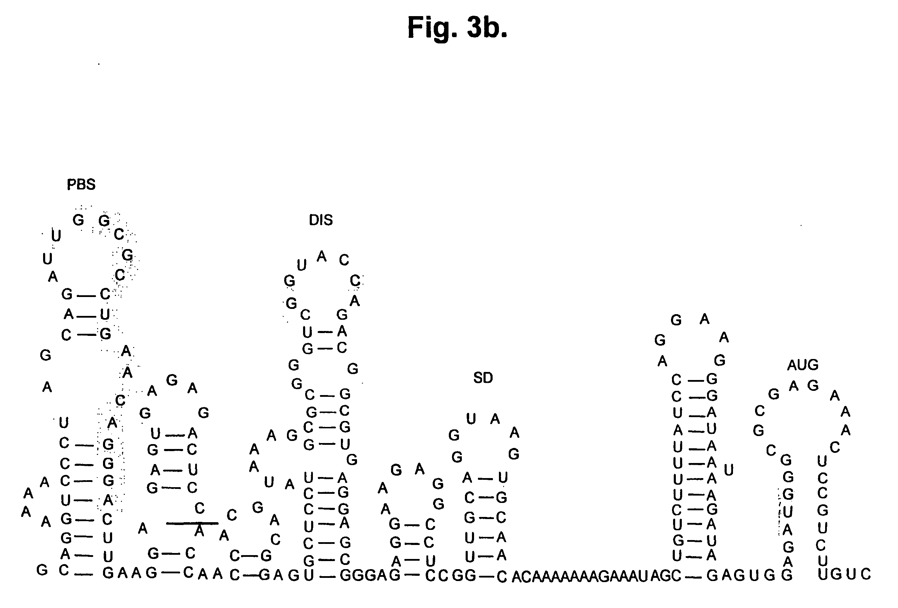

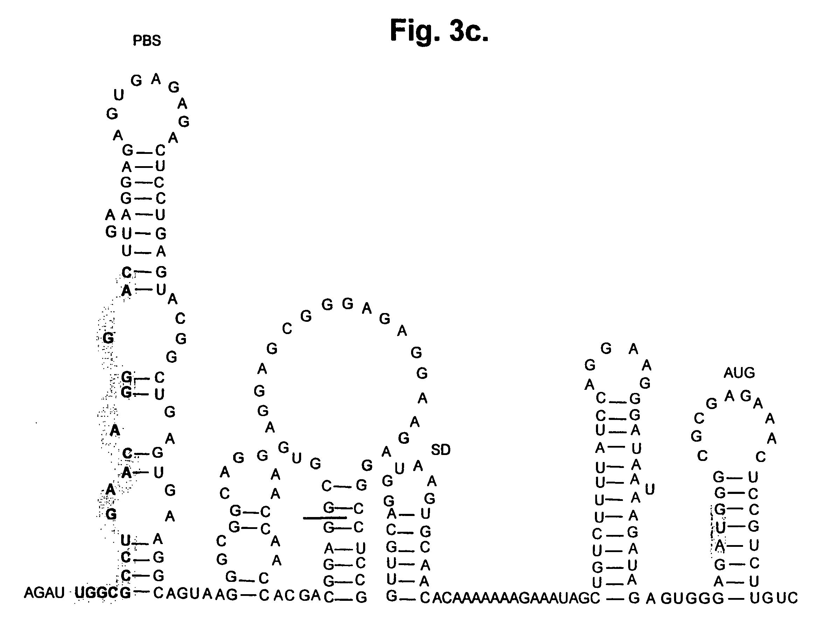

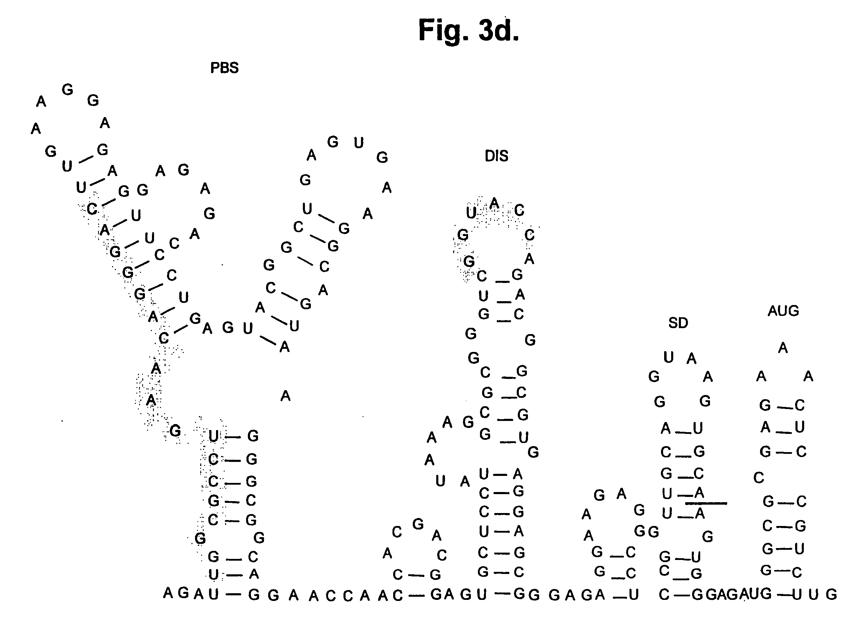

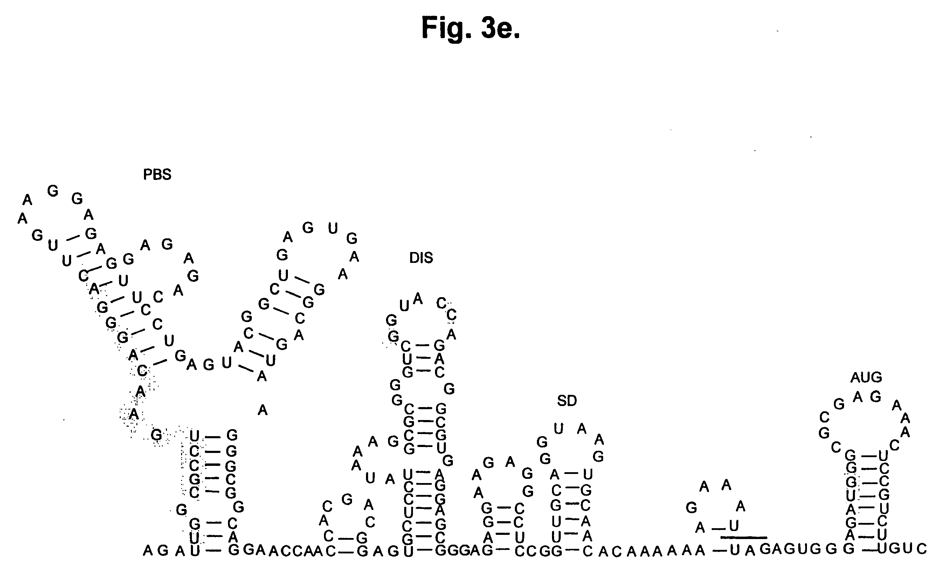

This region of the SIV genome is the structural fold termed DIS in the accompanying figure and is associated with a palindromic terminus. Preferably, the packaging sequences in this region are therefore mutated to disrupt the formation of the palindromic terminus and thus remove the DIS structure shown in Figure 3.

In another embodiment, other sequences, such as those downstream of the 5' major splice donor site extending up to the gag initiation codon are deleted. Preferably such sequences are deleted in addition to the mutation of sequences upstream of the major splice donor. For example, the virus genome has an additional deletion or mutation in the 50-base segment sequence shown as SEQ ID No. 2, i.e.
GAAATAGCTGTCTTGTTACCAGGAAGGGATAATAAGATAGATTGGGAGA T

The number of bases that need to be deleted or mutated can vary greatly. For example, the given 50 or 85-base pair deletions in SIV are sufficient to result in loss of packaging ability. However, even smaller deletions in this region could also result in loss of packaging efficiency. Indeed, it is expected that a deletion as small as about 5, 10, 15, 20 or 30 bases in this region and in particular a deletion in the region of nucleotides 53 to 85 of SEQ ID No 1, can remove efficient packaging ability. The size of a particular deletion can readily be determined based on the present disclosure by the person of ordinary skill in the art.

The vector should contain an SIV nucleotide segment containing a sufficient number of nucleotides corresponding to nucleotides of the SIV genome to express functional SIV gene products, but as described above, should not contain a sufficient number of nucleotides corresponding to the region between the primer-binding site and the 5' major splice donor or between 5' major splice donor and *gag* gene to permit efficient packaging of the viral RNA into virions. In using these vectors to establish SIV packaging-defective cell lines, it is preferred that such cell lines do not produce any infectious SIV. Although a cell line transformed by these packaging-defective deficient vectors would have low infectivity because the cells are packaging-defective, some RNA can still be packaged into the virion. Accordingly, it is preferable that the SIV nucleotide segment does not correspond to the entire SIV genome so that, if some of the viral RNA is packaged into the virion, what is packaged will not be replication-competent virus.

Preferably, a selected cell line is transformed using at least two different vectors, each containing a different portion of the SIV genome and also not containing the sequence necessary for viral packaging. Then, by cotransfecting a cell with each vector, the cell would still be able to express all the SIV structural and enzymatic proteins and produce virions. In one preferred embodiment, the or each vector does not contain sequences corresponding to an SIV LTR (long terminal repeat sequence) but contains sequences corresponding to a promoter region and/or another genome's polyadenylation sequences. Selection of particular promoters and polyadenylation sequences can readily be determined based upon the particular host cell. Preferably the LTR to which the sequences do not correspond is the 3'LTR.

In one preferred embodiment, one vector includes sequences permitting expression of SIV proteins upstream of *env* and the second vector permits expression of the remaining proteins. For example, one vector contains an SIV nucleotide segment corresponding to a sufficient number of nucleotides upstream of the *gag* initiation codon to the *env* gene sequence to express the 5'-most gene products. The other vector contains an SIV nucleotide segment corresponding to a sufficient number of nucleotides downstream of the *gag* gene sequence and including a functional *env* gene sequence. Such vectors can be chemically synthesised from the reported gene sequence of the SIV genome or derived from the many available SIV proviruses, by taking advantage of the known restriction endonuclease sites in these viruses by the skilled artisan based on the present disclosure.

Preferably, a different marker gene is added to each vector. Then, using a preselected cell line cotransfected with these different vectors, and by looking for a cell containing both markers, a cell that has been cotransfected with both vectors is found. Such a cell would be able to produce all of the SIV proteins. Although virions would be produced, the RNA corresponding to the entire viral sequences would not be packaged in these virions. One can use more than two vectors, if desired, e.g. a *gag*/*pol* vector, a protease vector and an *env* vector.

Retroviruses can in some cases be pseudotyped with the envelope glycoproteins of other viruses. Consequently, one can prepare a vector containing a sufficient number of nucleotides to correspond to an *env* gene from a different retrovirus. Preferably, the 5'LTR of this vector would be of the same genome as the *env* gene. Such a vector could be used instead of an SIV *env* packaging-defective vector, to create virions. By such a change, the resultant vector systems could be used in a wider host range or could be restricted to a smaller host range, e.g. using an HIV *env* gene vector which would restrict the cell range to those bearing the CD4 protein. Using a vesicular stomatitis virus or rabies virus envelope protein would make the vector tropic for many different cell types.

Virtually any cell line can be used. Preferably, a mammalian cell line is used, for example CV-1,Hela, Raji, SW480 or CHO.

In order to increase production of the viral cellular products, one could use a promoter other than the 5' LTR, e.g. by replacing the 5' LTR with a promoter that will preferentially express genes in CV-1 or HeLa cells. The particular promoter used can easily be determined by the person of ordinary skill in the art depending on the cell line used, based on the present disclosure.

In order to enhance the level of viral cellular products, one can also add enhancer sequences to the vector to get enhancement of the SIV LTR and/or promoter. Particular enhancer sequences can readily be determined by a person of ordinary skill in the art depending on the host cell line.

By using a series of vectors that together contain the complete SIV genome, one can create cell lines that produce a virion that is identical to the SIV virion except that the virion does not contain SIV RNA. These virions can readily be obtained from the cells. For example, the cells are cultured and the supernatant harvested. Depending on the desired use, the supernatant containing the virions can be used or these virions can be separated from the supernatant by standard techniques such as gradient centrifugation, filtering etc.

These attenuated virions are extremely useful in preparing a vaccine. The virions can be used to generate an antibody response to SIV virions and, because these virions are identical to the actual SIV virions except that the interior of these virions do not contain the viral RNA, the vaccine created should be particularly useful. Pseudotyped virions produced from cell lines cotransfected with SIV *gag*/*pol* and protease genes and containing the *env* gene from another virus may be useful in creating a vaccine-against this other virus. For example, an HIV *env* vector in the cell may give rise to a viral particle with an HIV *env* capable of eliciting an antibody response to HIV but without pathogenicity because of the absence of any other HIV proteins or HIV RNA.

These virions can also be used to raise antibodies to the virion that can then be used for a variety of purposes, e.g. screening for the virion, developing target systems for the virions etc. Additionally these SIV packaging-deficient cell lines can be extremely useful as a means of introducing a desired gene, for example a heterologous gene into mammalian cells, as described below.

In this aspect of the invention, a vector is provided which is capable of expressing a heterologous gene and additionally comprises the packaging sequences of SIV. Preferably the packaging sequences correspond to those described above which are mutated to produce a packaging defective SIV genome. Preferably a substantial portion of the packaging signal is included. In a preferred aspect the packaging sequence comprises the region upstream of the major 5' splice donor. In particular the packaging sequence is selected to allow the formation of a palindromic terminus, having the structure DIS shown in Figure 3. For example the packaging sequence of SEQ ID no 1 is provided, or a fragment thereof or a variant thereof, and in particular the region comprising nucleotides 53-85 of SEQ ID No 1. A variant thereof may be identified as set out above in determining a region of the genome to be deleted. All of the sequences described above for mutation or deletion to produce a SIV packaging defective genome are preferred sequences for incorporation into a vector such that the vector can be packaged by an SIV capsid or protein envelope. Preferably, the packaging sequences comprise a region of 10, 20, 30, 40, 50, 60, 70, 80, 100, 120, 150, 200 or more nucleotides upstream of the major 5' splice donor, and preferably include part or all of the sequence of SEQ ID No 1, or a corresponding sequence from a variant SIV genome. Additional sequences are also preferably provided such as 10, 20, 50, 70 or 100 or more polynucleotides from a region downstream of the 5' splice donor site up to the gag initiation codon, for example those of SEQ ID No 2.

These virions may be used as an extremely efficient way to package desired genetic sequences and deliver them to target cells infectable by SIV. This may be done by preparing a vector containing a nucleotide segment containing a sufficient number of nucleotides corresponding to the packaging nucleotides of SIV (SIV packaging region), a predetermined gene and, flanking the packaging sequence and predetermined gene, sequences corresponding to a sufficient number of sequences from within and near the LTR for packaging, reverse transcription, integration of the vector into target cells and gene expression from the vector.

The packaging region preferably corresponds to at least the region between the primer-binding site and the major 5' splice donor, as well as the region between the 5' major splice donor and the *gag* gene. With regard to the experimental data presented below concerning the packaging of such a vector, the vector might also have the first 500 bp of the *gag* gene sequence of SIV in order to enhance packaging efficiency. For example, a sufficient number of SIV sequences to be packaged, reverse-transcribed, integrated into and expressed in the target cells would include the U3,R and U5 sequences of the LTRs, the packaging sequences and some sequences flanking the LTRs (required for reverse transcription). Although the packaging sequences described between the primer-binding site and the 5' major splice donor and those between the major splice donor and the *gag* gene would be sufficient for packaging such a vector, it may be advantageous to include the first 500 nucleotides of the gag gene coding sequence as this appears to enhance packaging further. Mutation of the *gag* initiation codon would be acceptable to avoid translation starting from this point whilst still retaining the *cis* acting *gag* nucleotide sequence required for packaging. For example, the *gag* ATG may be changed to ATC by site-directed mutagenesis.

When this vector is used to transfect one of the SIV packaging-deficient cells, it is the nucleotide sequence from this vector that will be packaged in the virions. These SIV packaged genes may then be targeted to cells infectable by SIV. This method of transformation is expected to be much more efficient than current methods. Further, by appropriate choice of genes, the method of SIV infection may be monitored.

For example, the vector could contain a sufficient number of nucleotides corresponding to both 5' and 3' LTRs of SIV to be expressed, reverse-transcribed and integrated, a sufficient number of nucleotides corresponding to the SIV packaging sequence to be packaged. The vector would also contain a sufficient number of nucleotides of the gene which is desired to be transferred to produce a functional gene (e.g. gene segment). This gene can be any gene desired, as described below.

In an alternative aspect of the present invention the packaging sequences may be provided on their own or as antisense molecules to interfere with packaging of wild type SIV or to interfere with packaging of HIV in an HIV capsid. Thus the packaging sequences may be used in the prophylaxis or treatment of SIV or HIV infection, such as HIV-1 or HIV-2 infection and preferably HIV-1 infection. In particular packaging sequences such as those described either for deletion above or for incorporation with a vector for expression of heterologous genes may be used either alone or for the generation of antisense molecules as described in more detail below. The packaging sequences may be provided in a suitable delivery vehicle for example flanked by non-SIV or HIV sequences. Such packaging sequences can be used to bind to SIV or HIV capsid proteins or to saturate such binding sites or compete for such sites with wild type viral genome and thus prevent packaging of such genomes in the capsid. Thus the packaging sequences may be useful in therapy in their own right. Antisense molecules can be used to bind to wild type viral genome packaging sequences and thus prevent their recognition and binding with the viral capsid. Such packaging nucleotides may be formulated as described below or may be administered as naked polynucleotides or formulated with transfection facilitating agents as is well known in the art and delivered by any suitable technique.

Preferably the packaging sequences correspond to those described above which are mutated to produce a packaging defective SIV genome. A substantial portion of the packaging signal may be included. In a preferred aspect the packaging sequence comprises the region upstream of the major 5' splice donor. In particular the packaging sequence is selected to allow the formation of a palindromic terminus, having the structure DIS shown in Figure 3. For example the packaging sequence of SEQ ID no 1 is provided, or a fragment thereof or a variant thereof, and in particular the region comprising nucleotides 53-85 of SEQ ID No 1. A variant thereof may be identified as set out above in determining a region of the genome to be deleted. All of the sequences described above for mutation or deletion to produce a SIV packaging defective genome are preferred sequences for incorporation into a vector such that the vector can be packaged by an SIV capsid or protein envelope. Preferably, the packaging sequences comprise a region of 10, 20, 30, 40, 50, 60, 70, 80, 100, 120, 150, 200 or more nucleotides upstream of the major 5' splice donor, and preferably include part or all of the sequence of SEQ ID No 1, or a corresponding sequence from a variant SIV genome. Additional SIV sequences are also preferably provided such as 10, 20, 50, 70 or 100 or more polynucleotides from a region downstream of the 5' splice donor site up to the gag initiation codon, for example those of SEQ ID No 2. Alternatively or additionally, other flanking sequences may be provided for delivery of the packaging sequences. Antisense molecules which are complementary to the packaging sequences described herein may also be provided.

### Methods of mutation

The present invention relates to SIV genomes which are rendered packaging deficient, that is they are no longer packaged into the SIV capsid through mutation within the packaging sequences as discussed above. Such SIV genomes may also comprise other mutations within other SIV genes. Preferably such SIV genomes retain the ability to express and assemble the SIV capsid.

The invention additionally relates to a vector for expression of a heterologous gene which is packaged into the SIV genome through the use of SIV packaging sequences. Such a vector may comprise any suitable vector compatible with the proposed administration or use of the virus so long as the SIV packaging sequences are incorporated. Preferably the vector is derived from the SIV genome but includes mutation in one or more SIV genes, for example, to render the SIV genome replication deficient.

Essential genes may be rendered functionally inactive by several techniques well known in the art. For example, they may be rendered functionally inactive by deletions, substitutions or insertions, preferably by deletion. Deletions may remove portions of the genes or the entire gene. For example, deletion of only one nucleotide may be made, resulting in a frame shift. However, preferably larger deletions are made, for example at least 25%, more preferably at least 50% of the total coding and non-coding sequence (or alternatively, in absolute terms, at least 10 nucleotides, more preferably at least 100 nucleotides, most preferably, at least 1000 nucleotides). It is particularly preferred to remove the entire gene and some of the flanking sequences. Inserted sequences may include the heterologous genes described below.

Mutations are made in SIV by homologous recombination methods well known to those skilled in the art. For example, SIV genomic RNA is transfected together with a vector, preferably a plasmid vector, comprising the mutated sequence flanked by homologous SIV sequences. The mutated sequence may comprise deletions, insertions or substitutions, all of which may be constructed by routine techniques. Insertions may include selectable marker genes, for example lacZ, for screening recombinant viruses by, for example, β-galactosidase activity.

### Heterologous genes and promoters

A vector or viruses of the invention may be modified to carry a heterologous gene, that is to say a gene other than one present in the SIV genome. In particular the invention provides viral vectors which have SIV derived sequences sufficient to allow packaging of the vector into a SIV capsid. The vectors may otherwise be derived from SIV genomes, incorporating mutations or deletions in one or more SIV genes, or may be derived from other expression vectors which are modified to incorporate SIV packaging sequences. The term "heterologous gene" comprises any gene other than one present in the SIV genome. The heterologous gene may be any allelic variant of a wild-type gene, or it may be a mutant gene. The term "gene" is intended to cover nucleic acid sequences which are capable of being at least transcribed. Thus, sequences encoding mRNA, tRNA and rRNA are included within this definition. The sequences may be in the sense or antisense orientation with respect to the promoter. Antisense constructs can be used to inhibit the expression of a gene in a cell according to well-known techniques. Sequences encoding mRNA will optionally include some or all of 5' and/or 3' transcribed but untranslated flanking sequences naturally, or otherwise, associated with the translated coding sequence. It may optionally further include the associated transcriptional control sequences normally associated with the transcribed sequences, for example transcriptional stop signals, polyadenylation sites and downstream enhancer elements.

The heterologous gene may be inserted into for example an SIV genome by homologous recombination of SIV strains with, for example, plasmid vectors carrying the heterologous gene flanked by SIV sequences. The heterologous gene may be introduced into a suitable plasmid vector comprising SIV sequences using cloning techniques well-known in the art. The heterologous gene may be inserted into the SIV genome or vector at any location. It is preferred that the heterologous gene is inserted into an essential SIV gene. Preferably the vector is derived from an SIV genome, but includes deletion of one, two or several of the SIV genes, up to the minimal sequences of the SIV genome to provide for packaging and expression of the heterologous gene.

The transcribed sequence of the heterologous gene is preferably operably linked to a control sequence permitting expression of the heterologous gene in mammalian cells. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence.

The control sequence comprises a promoter allowing expression of the heterologous gene and a signal for termination of transcription. The promoter is selected from promoters which are functional in mammalian, preferably human, cells. The promoter may be derived from promoter sequences of eukaryotic genes. For example, it may be a promoter derived from the genome of a cell in which expression of the heterologous gene is to occur. With respect to eukaryotic promoters, they may be promoters that function in a ubiquitous manner (such as promoters of β-actin, tubulin) or, alternatively, a tissue-specific manner (such as promoters of the genes for pyruvate kinase). They may also be promoters that respond to specific stimuli, for example promoters that bind steroid hormone receptors. Viral promoters may also be used, for example the Moloney murine leukaemia virus long terminal repeat (MMLV LTR) promoter or promoters of SIV genes.

The SIV LTR promoter, and promoters containing elements of the LTR promoter region, are especially preferred. The expression cassette may further comprise a second promoter and a second heterologous gene operably linked in that order and in the opposite or same orientation to the first promoter and first heterologous gene wherein said second promoter and second heterologous gene are the same as or different to the first promoter and first heterologous gene. Thus a pair of promoter/heterologous gene constructs may allow the expression of pairs of heterologous genes, which may be the same or different, driven by the same or different promoters. Furthermore, the product of the first heterologous gene may regulate the expression of the second heterologous gene (or vice-versa) under suitable physiological conditions.

The expression cassette can be constructed using routine cloning techniques known to persons skilled in the art (see, for example, Sambrook *et al.,* 1989, Molecular Cloning - a laboratory manual; Cold Spring Harbor Press).

It may also be advantageous for the promoters to be inducible so that the levels of expression of the heterologous gene can be regulated during the life-time of the cell. Inducible means that the levels of expression obtained using the promoter can be regulated. For example, in a preferred embodiment where more than one heterologous gene is inserted into the vector or SIV genome, one promoter would comprise a promoter responsive to the expression of the second protein and driving the heterologous gene the expression of which is to be regulated. The second promoter would comprise a strong promoter (e.g. the CMV IE promoter) driving the expression of the second protein.

In addition, any of these promoters may be modified by the addition of further regulatory sequences, for example enhancer sequences. Chimeric promoters may also be used comprising sequence elements from two or more different promoters described above, for example an MMLV LTR/SIV fusion promoter.

The heterologous gene may encode, for example, proteins involved in the regulation of cell division, for example mitogenic growth factors, cytokines (such as α-, β- or γ-interferon, interleukins including IL-1, IL-2, tumour necrosis factor, or insulin-like growth factors I or II), protein kinases (such as MAP kinase), protein phosphatases and cellular receptors for any of the above. The heterologous gene may also encode enzymes involved in cellular metabolic pathways, for example enzymes involved in amino acid biosynthesis or degradation (such as tyrosine hydroxylase), or protein involved in the regulation of such pathways, for example protein kinases and phosphatases. The heterologous gene may also encode transcription factors or proteins involved in their regulation, membrane proteins (such as rhodopsin), structural proteins (such as dystrophin) or heat shock proteins such as hsp27, hsp65, hsp70 and hsp90.

Preferably, the heterologous gene encodes a polypeptide of therapeutic use, or whose function or lack of function may be important in a disease process. For example, tyrosine hydroxylase can be used in the treatment of Parkinson's disease, rhodopsin can be used in the treatment of eye disorders, dystrophin may be used to treat muscular dystrophy, and heat shock proteins can be used to treat disorders of the heart and brain associated with ischaemic stress. Polypeptides of therapeutic use may also include cytotoxic polypeptides such as ricin, or enzymes capable of converting a precursor prodrug into a cytotoxic compound for use in, for example, methods of virus-directed enzyme prodrug therapy or gene-directed enzyme prodrug therapy. In the latter case, it may be desirable to ensure that the enzyme has a suitable signal sequence for directing it to the cell surface, preferably a signal sequence that allows the enzyme to be exposed on the exterior of the cell surface whilst remaining anchored to cell membrane.

Heterologous genes may also encode antigenic polypeptides for use as vaccines. Preferably such antigenic polypeptides are derived from pathogenic organisms, for example bacteria or viruses, or from tumours.

Heterologous genes may also include marker genes (for example encoding β-galactosidase or green fluorescent protein) or genes whose products regulate the expression of other genes (for example, transcriptional regulatory factors.

Gene therapy and other therapeutic applications may well require the administration of multiple genes. The expression of multiple genes may be advantageous for the treatment of a variety of conditions.

### Host cells

In one aspect of the present invention, host cells are generated to produce SIV virus containing a vector for expression of a heterologous gene. The viruses are produced by co-transfecting a cell with a packaging defective SIV genome according to the invention which is capable of producing an SIV capsid and a vector according to the invention having an SIV packaging signal and a heterologous gene. Such viruses are produced by co-transfecting a suitable cell such as a mammalian cell with both vectors.

### Administration

The viruses of the present invention may thus be used to deliver therapeutic genes to a human or animal in need of treatment.

One method for administered gene therapy involves inserting the therapeutic gene into a vector of the invention, as described above. Subsequently, cells are cotransfected in vitro with a vector comprising the heterologous gene and the SIV packaging sequences and a packaging defective SIV genome. Culturing the cells leads to production of SIV viral capsids, into which the heterologous gene vectors are packaged through the SIV packaging sequences. The resultant recombinant virus may be combined with a pharmaceutically acceptable carrier or diluent to produce a pharmaceutical composition. Suitable carriers and diluents include isotonic saline solutions, for example phosphate-buffered saline. Vaccine compositions, in which the heterologous gene encodes an antigenic peptide or protein may be formulated with adjuvants to enhance the immune response generated. The composition may be formulated for parenteral, intramuscular, intravenous, subcutaneous, intraocular or transdermal administration.

The pharmaceutical composition is administered in such a way that the virus containing the therapeutic gene for gene therapy, can be incorporated into cells at an appropriate area. The SIV capsids containing the heterologous gene constructs are particularly useful due to the ability of SIV to infect non dividing cells of many different types. In addition SIV is non-pathogenic to humans and is particularly useful for therapy or vaccination.

The amount of virus administered is in the range of from 10⁴ to 10¹⁰ pfu, preferably from 10⁵ to 10⁸ pfu, more preferably about 10⁶ to 10⁷ pfu. When injected, typically 1 to 10 µl of virus in a pharmaceutically acceptable suitable carrier or diluent is administered.

The routes of administration and dosages described are intended only as a guide since a skilled practitioner will be able to determine readily the optimum route of administration and dosage for any particular patient and condition.

### Assay Methodologies

The viruses of the invention can also be used in methods of scientific research. Thus, a further aspect of the present invention relates to methods of assaying gene function in mammalian cells, either *in vitro* or *in vivo.* The function of a heterologous gene could be determined by a method comprising:
(a) producing virus particles comprising an SIV capsid and vector having a heterologous gene packaged via SIV packaging signals, and
(b) introducing the resulting virus into a mammalian cell line; and
(c) determining the effect of expression of said heterologous gene in said mammalian cell-line.

For example, the cell-line may have a temperature-sensitive defect in cell division. When an SIV strain comprising a heterologous gene according to the invention is introduced into the defective cell-line and the cell-line grown at the restrictive temperature, a skilled person will easily be able to determine whether the heterologous gene can complement the defect in cell division. Similarly, other known techniques can be applied to determine if expression of the heterologous gene can correct an observable mutant phenotype in the mammalian cell-line.

This procedure can also be used to carry out systematic mutagenesis of a heterologous gene to ascertain which regions of the protein encoded by the gene are involved in restoring the mutant phenotype.

This method can also be used in animals, for example mice, carrying so-called "gene knock-outs". A wild-type heterologous gene can be introduced into the animal using a mutant SIV strain of the invention and the effect on the animal determined using various behavioural, histochemical or biochemical assays known in the art. Alternatively, a mutant heterologous gene can be introduced into either a wild-type or "gene knock-out" animal to determine if disease-associated pathology is induced. An antisense nucleotide could also be introduced using the virus particle of the invention to create in effect a knock-out animal.

Alternatively, the mutant SIV virus of the invention may be used to obtain expression of a gene under investigation in a target cell with subsequent incubation with a test substance to monitor the effect of the test substance on the target gene.

Thus, the methods of the present invention may be used in particular for the functional study of genes implicated in disease.

The invention will be described with reference to the following Example, which are intended to be illustrative only and not limiting.

### Example

In summary, the infectious proviral clone has been described in publications including those by Rud. It comprises a complete proviral clone in a pBluescript KS-backbone. Using oligonucleotide site-directed mutagenesis, a series of segments in the non-coding region has now been deleted between primer binding site and the *gag* initiation codon. Deletion ΔP1 includes nucleotides 171-207, ΔP2 224-256, ΔP3 304-354 and ΔP4 320-351. All of these nucleotide numberings refer to the RNA of the virus where the first base in the R region is considered +1. The proviral clone also had a reporter gene construct substituted into the envelope region for detection purposes. The constructs were transfected into Cos-1 cells and the RNA present in the cells in the virions analysed by the highly sensitive quantitative and qualitative RNase protection assay. A reduction in the production of particles was seen with all of the deletions. Those causing the most severe defects were ΔP3 and ΔP4, reducing packaging to approximately 20% of wild type. The deletions ΔP1 and ΔP2 reduced packaging to approximately 30% of that of wild type. Thus, the SIV leader region appears to have a packaging signal which is distributed widely across the long section of the leader between the primer-binding site and the *gag* initiation codon. The major packaging signal lies upstream of the major 5' splice donor. In this respect, it differs from previously documented lentiviral packaging signals. In HIV-1, virtually the whole of the packaging specificity is found between the splice donor and the *gag* initiation codon, deletions here leading to a reduction in packaging of over 90% (Lever *et al*). In HIV-2, the major packaging signal is found almost exclusively upstream of the splice donor, between the primer-binding site and the major 5' splice donor (McCann and Lever). Confirmation is thus obtained that it is not possible to predict the exact site or size of the packaging signals in lentiviruses from prior experimentation. The present disclosure allows someone skilled in the art to construct an efficient retroviral vector system based on SIV.

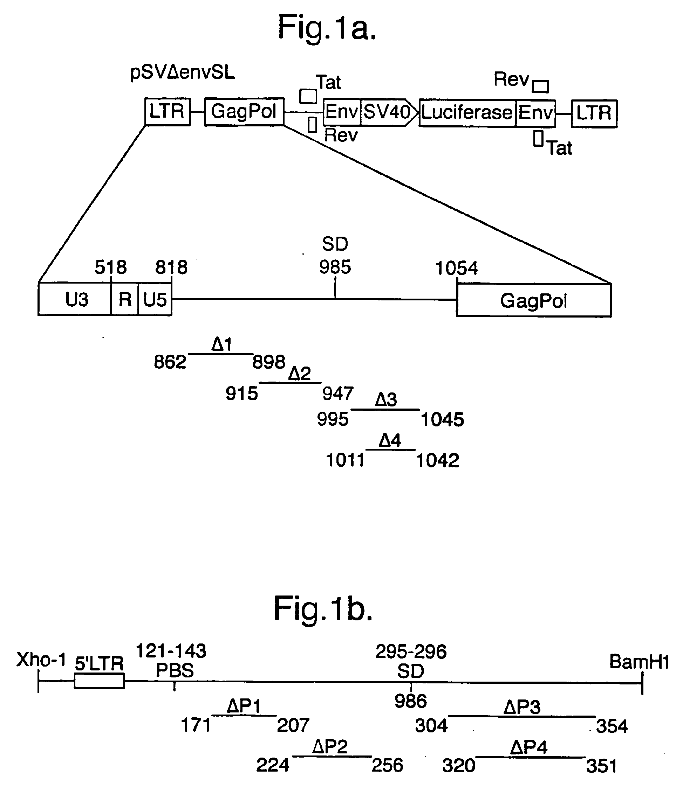

To study SIV in more detail, a series of SIV-based vectors were constructed based on the C8 clone of the SIV_{mac239} (Rud et al 1994 J. Gen Virol 75, 529-543) which contains a mutation in the Nef open reading frame associated with an attenuated phenotype. A deletion was made in the envelope open reading frame and, into this, an expression cassette consisting of the firefly luciferase gene expressed from the SV40 early promoter was inserted between the *PflM* 1 and *Pml*1 sites (Figure 1). Confirmation that this construct pRSΔenvSL was capable of producing reverse transcriptase and viral particles and encapsidating its RNA was achieved by co-transfecting this construct with either the SIV envelope, the amphotropic murine leukaemia virus envelope or the VSV-G envelope protein. All of these experiments led to gene transfer and expression of the luciferase gene in target cell lines.

The 5' segment of the provirus was cloned into a separate plasmid, pBluescript KS, and site-directed mutagenesis was carried out using the Kunkel method (Kunkel *et al*, 1987 Meth Enzymol, 154,367-82).

For Δ1, positions 862-898 (numbering from the start of the 5' U3 sequence, or 171-207 when the first base of the R region is numbered 1) were deleted using the mutagenic oligonucleotide 5' AGTGAGAAGAACTCCACCACGACGGACTGC 3'.

For Δ2, positions 915-947 (or 224-256 on alternative numbering) were deleted using the mutagenic oligonucleotide 5' CCAACCACGACGGAGGCGTGAGGAGCG 3'.

For Δ3, positions 995-1045 (or 304-354 on alternative numbering) were deleted using the mutagenic oligonucleotide 5' CGGTTGCAGGTAAGTGCAAGTGGGAGATGGGC 3'.

For Δ4, positions 1011-1042 (or 320-351) were deleted using the mutagenic oligonucleotide 5' GCAACACAAAAAAAGAGTGGGAGATGGGC 3'.

The deleted 5' leader sequences were recloned back individually into the SIV gag/pol reporter construct, pRS ΔenvSL (Figure 1) generating four new plasmids Δ1-4 ΔenvSL.

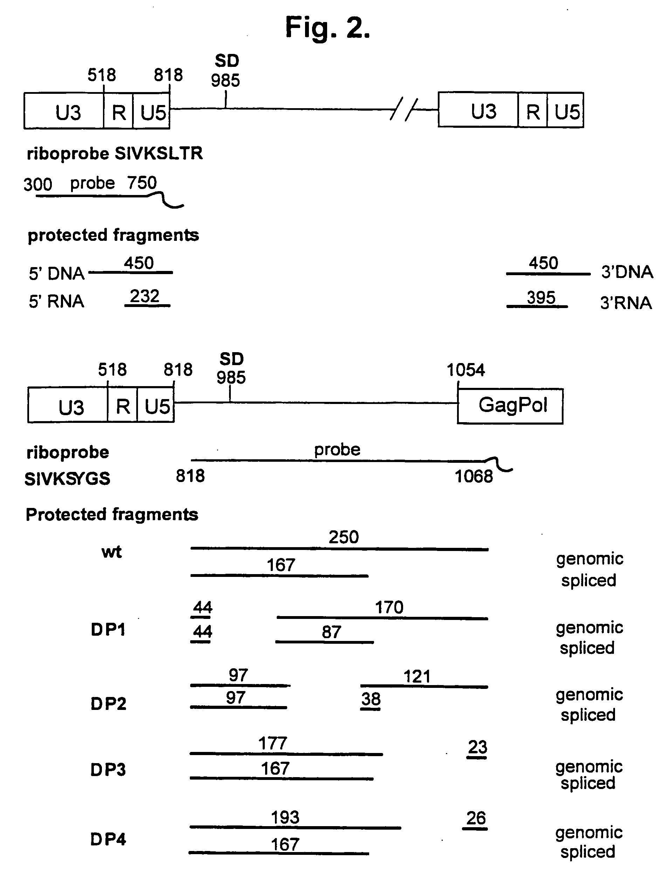

Plasmids used as templates for the production of riboprobes were created as follows: SIVKSΨGS used to detect genomic versus spliced RNA was created by amplification of SIV sequences between 818 and 1068 using the primers 5' ATGGGAATTCGTTTCGTTTCTCGCGCCCATCTCCCACTCT 3' and 5'TAATGGATCCAGATTGGCGCCTGAACAGGG 3'. The PCR product was then cloned into the *Bam* H1 and *Eco* R1 sites of Bluescript SK + (Stratagene). SIVKSLTR used to discriminate DNA from RNA was created by amplification of SIV sequences between 300 and 750 using the primers 5' CTTTGAATTCACCGAGTACCGAGTTG 3' and 5' TTTGGGATCCTACCCAGAAGAGTTTGG 3' (Figure 2). The PCR product was then cloned into the *Bam* H1 and *Eco* R1 sites of Bluescript SK + (Stratagene).

pRS ΔenvSL containing either the wild type leader sequence or the deletion mutants (Figure 1) were transfected into 293T cells which had been maintained in MEM supplemented with 10% fetal calf serum, 100 µg/ml streptomycin and 100 U/ml penicillin. Transient transfections were performed with 10 µg plasmid using a modified calcium phosphate technique. Cells and supernatants were harvested 48 hours post-transfection.

Wild type and mutant SIV vectors were assessed for protein production by immunoprecipitation as previously described (McCann and Lever 1997, J. Virol. 71, 4133-4137) using polyclonal anti-SIV antisera. In particular, immunoprecipitation of viral core proteins from supernatants of cells transfected with proviral constructs was carried out. Wild type lane contained two fold less virus to avoid overloading.

In wild type, Δ1, Δ2 and Δ3 SIV specific bands representing the major structural proteins of SIV were detectable. Surprisingly, the smaller nested deletion Δ4 showed no viral protein in transfected cells.

Packaging of SIV RNA was assessed by RNase protection assays as previously described and the ratio of genomic to spliced RNA used in a previous publications to calculate a packaging efficiency (Richardson et al. 1993 J. Virol 67, 3997-4005) using mean values from replicate RNase protection assays. The virion lanes were normalised for reverse transcriptase in the case of wild type, Δ1, Δ2 and Δ3. For Δ4 the value was very low and, thus, the complete supernatant sample was tested. Efficient DNA removal from the RNA was confirmed using the riboprobe SIVKSLTD (Figure 2). Virions and cellular RNA bands using riboprobe SIVKSΨGS were analysed. RNase protection of SIV RNA from cells transfected with constructs and virions from cell supernatants. LTR specific probe demonstrates specificity of signal for RNA in that for WT virus only bands of 395 and 232 are detected. No DNA specific band at 450 is seen. Results are representative of three replicate experiments. There was an absence of signal from Δ4 virion lane other than background bands also seen in adjacent Δ3, mock and Probe + RNase lanes. The results summarised in Table 1.

**TABLE 1**

| *plasmid* | *ratio of spliced: genomic* | | *packaging efficiency relative to wild type* |
|---|---|---|---|
| | *Cytoplasmic* | *virion* | |
| Wild type | 6.4 | 0.1 | 1 |
| Δ1 | 2.5 | 1.7 | 0.16 |
| Δ2 | 3.1 | - | 0 |
| Δ3 | 2.7 | 0.27 | 0.5 |
| Δ4 | 5 | - | 0 |

Of interest is the high proportion of spliced viral RNA detectable in the cells. This is greater than we have previously observed for HIV-1 or HIV-2. It can be seen that the wild type RNA packages itself with a high level of specificity and there is a small but detectable amount of packaged spliced virus RNA. Deletions Δ1 and more particularly Δ2 lead to a reduction in the quantity of full-length genomic RNA packaged whilst having little effect on the relative proportions of full-length and spliced RNA found in the cytoplasm of the transfected cells. Δ1 also appears to enhance protein production as we have previously noted in some HIV leader region deletion mutants. In the case Δ2, genomic RNA is virtually undetectable in the virions. This result was reproducible in all the experiments. The Δ3 deletion which is the larger of the two deletions 3' to the major splice donor has overall a lower level of genomic and spliced RNA in the cytoplasm. However, there is selectivity in that the genomic RNA is encapsidated at a much higher level than the spliced RNA at a ratio similar to wild type virus. Despite the appearance of reasonable quantities of genomic unspliced and spliced RNA in the cytoplasm of cells transfected with Δ4 mutant, we were unable, on repeated occasions, to detect a signal consistent with the appearance of packaged RNA from transfected cell supernatants. A faint band of approximately the correct size for spliced RNA was occasionally seen. However, this same band was always found in the control lane. Repeated sequencing of the leader region and the entire *gag* gene of Δ4 revealed no additional sequence mutations other than the introduced deletion.

The results from the replicates of the ribonuclease protection assays are presented in Table 1 and show that the deletion Δ2 correlates most closely with removal of a region that would be characteristic of an important *cis*-acting packaging signal. The ratio of genomic to spliced RNA in the cytoplasm is similar to that seen in the mutant and WT viruses. However, there is a virtual absence of genomic RNA in the virion. The deletions Δ1 and Δ3 permit RNA encapsidation although, in the case of Δ3, there is some reduction in the global quantity of RNA detectable in the virion. These results would be most consistent with the major packaging signal of SIV existing upstream of the major splice donor.

The phenotype of the Δ4 mutant was something of a puzzle since this deletion is nested within the span of the Δ3 deletion and, if anything, would be expected to have less of an effect on *cis*-acting functions. In fact, the striking feature of this mutant was the apparent absence of viral protein production despite abundant viral RNA in the cell. No additional sequence mutations were found in Δ4 to explain the lack of Gag protein.

We speculated as to whether the Δ4 mutation might have a more profound effect on the secondary structure of the leader RNA and investigated this using the RNA folding programme (www.ibc.wustl.edu/~zuker/rna/). The results are shown in Figure 3 (a-e). Figure 3 shows the RNA structure of the intact region in part a, on which are marked the boundaries of 4 deletion mutants that have been created: at the most extreme 5' end, there is a deletion between the two arrows marked ΔP1; in b, the resulting structure with a bar marks the site of the deleted sequence. The same is true for c for ΔP2 which clearly deletes a structure labelled DIS with some shaded bases (GGUACC) at the tip. Of interest was the fact that the Δ2 mutation which caused the profound packaging defect deletes an RNA stem loop with a palindromic terminus which would be consistent with part of the leader involved in dimerisation and encapsidation. This remains intact in the Δ1, Δ3 and Δ4 deletions. These deletions did not cause a packaging deletion indicating that the packaging signal has been precisely identified. The Δ4 deletion, however, significantly disrupts the structured region between the putative packaging signal/dimer initiation signal loop and the stem loop containing the viral *gag* initiation codon. Although the Δ3 mutation also disrupts this region, the change in predicted secondary structure is not as severe as that brought about by Δ4 in which a stem loop present in the wild type and other mutants upstream of the Gag initiation codon is replaced by a region of unstructured RNA. We suggest that the dramatic effect of Δ4 may be caused by the severe disruption of secondary structure, possibly affecting *cis* acting functions other than packaging which are dependent on this region.

## Claims

1. A Simian Immunodeficiency Virus (SIV) genome having a mutation within the packaging signal such that viral RNA is not packaged within an SIV capsid, wherein said mutation comprises deletion of
(a) a sequence of SEQ ID no 1, or
(b) a fragment thereof of 5 or more nucleotides in length, or
(c) a variant of either thereof.

2. An SIV genome according to claim 1 wherein the genome has a deletion in the region between the primer binding site and the 5' major splice donor site.

3. An SIV genome according to claim 1 or 2 wherein the genome comprises a mutation in the region between the 5' major splice donor site and the *gag* initiation codon.

4. An SIV genome according to any one of the preceding claims wherein the genome has a mutation within the DIS structure.

5. A SIV genome according to claim 1 wherein the deletion comprises nucleotides 53-85 of SEQ ID No 1.

6. A viral vector comprising an SIV packaging signal and a heterologous gene capable of being expressed in the vector, wherein the SIV packaging signal is
(a) a sequence of SEQ ID no 1, or
(b) a fragment thereof of 10 or more nucleotides in length, or
(c) a variant of either thereof.

7. A vector according to claim 6 comprising the region between the primer binding site and the 5' major splice donor site, and/or the region between the 5' major splice donor site and the *gag* initiation codon or a fragment of either thereof.

8. A vector according to claim 6 or 7 wherein the heterologous gene encodes a therapeutic protein or peptide, an antigen protein or peptide.

9. A process for producing a SIV virus encoding a heterologous gene, which process comprises infecting a host cell with a packaging defective SIV genome according to any one of claims 1 to 5 and a viral vector according to any one of claims 6 to 8; and culturing the host cell.

10. A pharmaceutical composition comprising a virus produced by a process according to claim 9 and a pharmaceutically acceptable carrier.

11. An SIV packaging sequence or an antisense sequence thereto, for use in the treatment or prophylaxis of SIV or HIV infection, wherein said SIV packaging sequence comprises:
(a) a sequence of SEQ ID no 1, or
(b) a fragment thereof of 5 or more nucleotides in length, or
(c) a variant of either thereof.

12. An SIV packaging sequence according to claim 11 comprising a sequence of 5 or more polynucleotides from a region of the SN genome between the primer binding site and the major 5' splice donor.

13. An SIV packaging sequence according to claim 11 or 12 for use in the manufacture of a medicament for the treatment or prophylaxis of SIV or HIV infection.

## Patentansprüche

1. Simian-Immunschwäche-Virus (SIV)-Genom mit einer Mutation innerhalb des Verpackungssignals, so dass die virale RNA nicht in einem SIV-Capsid verpackt ist, wobei die Mutation die Deletion von
(a) einer Sequenz von SEQ ID NO: 1 oder
(b) einem Fragment davon mit einer Länge von 5 oder mehr Nucleotiden oder
(c) einer Variante von einem der beiden
umfasst.

2. SIV-Genom nach Anspruch 1, wobei das Genom eine Deletion in der Region zwischen der Primerbindungsstelle und der 5'-Hauptspleiß-Donatorstelle aufweist.

3. SIV-Genom nach Anspruch 1 oder 2, wobei das Genom eine Mutation in der Region zwischen der 5'-Hauptspleiß-Donatorstelle und dem gag-Initiationscodon umfasst.

4. SIV-Genom nach einem der vorstehenden Ansprüche, wobei das Genom eine Mutation innerhalb der DIS-Struktur aufweist.

5. SIV-Genom nach Anspruch 1, wobei die Deletion die Nucleotide 53-85 von SEQ ID NO: 1 umfasst.

6. Viraler Vektor, umfassend ein SIV-Verpackungssignal und ein heterologes Gen, das zur Expression im Vektor befähigt ist, wobei es sich beim SIV-Verpackungssignal um
(a) eine Sequenz von SEQ ID NO: 1 oder
(b) ein Fragment davon mit einer Länge von 10 oder mehr Nucleotiden oder
(c) eine Variante von einem der beiden
handelt.

7. Vektor nach Anspruch 6, umfassend die Region zwischen der Primerbindungsstelle und der 5'-Hauptspleiß-Donatorstelle und/oder die Region zwischen der 5'-Hauptspleiß-Donatorstelle und dem gag-Initiationscodon oder ein Fragment von einem der beiden.

8. Vektor nach Anspruch 6 oder 7, wobei das heterologe Gen für ein therapeutisches Protein oder Peptid, ein Antigenprotein oder -peptid kodiert.

9. Verfahren zur Herstellung eines SIV-Virus, das für ein heterologes Gen kodiert, wobei das Verfahren das Infizieren einer Wirtszelle mit einem verpackungsdefektiven SIV-Genom nach einem der Ansprüche 1 bis 5 und einem viralen Vektor nach einem der Ansprüche 6 bis 8; und das Züchten der Wirtszelle umfasst.

10. Pharmazeutische Zusammensetzung, umfassend ein Virus, das gemäß einem Verfahren nach Anspruch 9 hergestellt worden ist, und einen pharmazeutisch verträglichen Trägerstoff.

11. SIV-Verpackungssequenz oder eine antisense-Sequenz hierzu zur Verwendung bei der Therapie oder Prophylaxe einer SIV- oder HIV-Infektion, wobei die SIV-Verpackungssequenz folgendes umfasst:
(a) eine Sequenz von SEQ ID NO: 1 oder
(b) ein Fragment davon mit einer Länge von 5 oder mehr Nucleotiden oder
(c) eine Variante von einem der beiden.

12. SIV-Verpackungssequenz nach Anspruch 11, umfassend eine Sequenz mit 5 oder mehr Polynucleotiden aus einer Region des SIV-Genoms zwischen der Primerbindungsstelle und der 5'-Hauptspleiß-Donatorstelle.

13. SIV-Verpackungssequenz nach Anspruch 11 oder 12 zur Verwendung bei der Herstellung eines Arzneimittels zur Therapie oder Prophylaxe einer SIV- oder HIV-Infektion.

## Revendications

1. Génome du virus d'immunodéficience simienne (SIV) ayant une mutation dans le signal d'encapsidation telle que l'ARN viral ne soit pas encapsidé dans une capside de SIV, ladite mutation comprenant la délétion
(a) d'une séquence de la SEQ ID N° 1, ou
(b) d'un de ses fragments ayant une longueur de 5 ou plus de 5 nucléotides, ou
(c) d'un variant de cette séquence ou de ce fragment.

2. Génome de SIV suivant la revendication 1, le génome ayant une délétion dans la région entre le site de liaison d'amorce et le site donneur d'épissage principal 5'.

3. Génome de SIV suivant la revendication 1 ou 2, le génome comprenant une mutation de la région entre le site donneur d'épissage principal 5' et le codon d'initiation *gag.*

4. Génome de SIV suivant l'une quelconque des revendications précédentes, le génome ayant une mutation dans la structure DIS.

5. Génome de SIV suivant la revendication 1, dans lequel la délétion comprend les nucléotides 53 à 85 de la SEQ ID N° 1.

6. Vecteur viral comprenant un signal d'encapsidation de SIV et un gène hétérologue capable d'être exprimé dans le vecteur, dans lequel le signal d'encapsidation de SIV est
(a) une séquence de la SEQ N° 1, ou
(b) un de ses fragments ayant une longueur de 10 ou plus de 10 nucléotides, ou
(c) un variant de cette séquence ou de ce fragment.

7. Vecteur suivant la revendication 6, comprenant la région entre le site de liaison d'amorce et le site donneur d'épissage principal 5' et/ou la région entre le site donneur d'épissage principal 5' et le codon d'initiation *gag* ou un fragment de l'une ou l'autre de celles-ci.

8. Vecteur suivant la revendication 6 ou 7, dans lequel le gène hétérologue code pour une protéine thérapeutique ou un peptide thérapeutique, une protéine antigénique ou un peptide antigénique.

9. Procédé pour la production d'un virus SIV codant pour un gène hétérologue, procédé qui comprend l'infection d'une cellule hôte avec génome de SIV à encapsidation défective suivant l'une quelconque des revendications 1 à 5 et un vecteur viral suivant l'une quelconque des revendications 6 à 8 ; et la culture de la cellule hôte.

10. Composition pharmaceutique comprenant un virus produit par un procédé suivant la revendication 9 et un support pharmaceutiquement acceptable.

11. Séquence d'encapsidation de SIV ou séquence anti-sens par rapport à celle-ci destinée à être utilisée dans le traitement ou la prophylaxie d'une infection par le SIV ou le HIV, ladite séquence d'encapsidation de SIV comprenant :
(a) une séquence de la SEQ ID N° 1, ou
(b) un fragment de celle-ci ayant une longueur de 5 ou plus de 5 nucléotides, ou
(c) un variant de cette séquence ou de ce fragment.

12. Séquence d'encapsidation de SIV suivant la revendication 11, comprenant une séquence de 5 ou plus de 5 polynucléotides d'une région du génome du SIV entre le site de liaison en amorce et le site d'épissage principal 5'.

13. Séquence d'encapsidation de SIV suivant la revendication 11 ou 12, destinée à être utilisée dans la production d'un médicament destiné au traitement ou à la prophylaxie d'une infection par le SIV ou le HIV.


### SEQUENCE LISTING

<110> SYNGENIX LIMITED
<120> VIRAL VECTORS
<130> N79496B GCW SER
<140>
   <141>
<150> GB 9913459.5
   <151> 1999-06-09
<150> GB 9916911.2
   <151> 1999-07-19
<160> 10
<170> PatentIn Ver. 2.1
<210> 1
   <211> 84
   <212> DNA
   <213> Simian immunodeficiency virus
<400> 1 
agaactcctg agtacggcct gagtgaaggc agtaagggcg gcaggaacca accacgacgg 60
agtgctccta taaaggcgca ggtcg 85
<210> 2
   <211> 50
   <212> DNA
   <213> Simian immunodeficiency virus
<400> 2
   gaaatagctg tcttgttacc aggaagggat aataagatag attgggagat 50
<210> 3
   <211> 30
   <212> DNA
   <213> Artificial Sequence
<220>
   <223> Description of Artificial Sequence: synthetic oligonucleotide
<400> 3
   agtgagaaga actccaccac gacggactgc 30
<210> 4
   <211> 27
   <212> DNA
   <213> Artificial Sequence
<220>
   <223> Description of Artificial Sequence: synthetic oligonucleotide
<400> 4
   ccaaccacga cggaggcgtg aggagcg 27
<210> 5
   <211> 32
   <212> DNA
   <213> Artificial Sequence
<220>
   <223> Description of Artificial Sequence: synthetic oligonucleotide
<400> 5
   cggttgcagg taagtgcaag tgggagatgg gc 32
<210> 6
   <211> 29
   <212> DNA
   <213> Artificial Sequence
<220>
   <223> Description of Artificial Sequence: synthetic oligonucleotide
<400> 6
   gcaacacaaa aaaagagtgg gagatgggc 29
<210> 7
   <211> 40
   <212> DNA
   <213> Artificial Sequence
<220>
   <223> Description of Artificial Sequence: synthetic oligonucleotide
<400> 7
   atgggaattc gtttcgtttc tcgcgcccat ctcccactct 40
<210> 8
   <211> 30
   <212> DNA
   <213> Artificial Sequence
<220>
   <223> Description of Artificial Sequence: synthetic oligonucleotide
<400> 8
   taatggatcc agattggcgc ctgaacaggg 30
<210> 9
   <211> 26
   <212> DNA
   <213> Artificial Sequence
<220>
   <223> Description of Artificial Sequence: synthetic oligonucleotide
<400> 9
   ctttgaattc accgagtacc gagttg 26
<210> 10
   <211> 27
   <212> DNA
   <213> Artificial Sequence
<220>
   <223> Description of Artificial Sequence: synthetic oligonucleotide
<400> 10
   tttgggatcc tacccagaag agtttgg 27